## Europäisches Patentamt

## European Patent Office

## Office européen des brevets

(19)

(11) Veröffentlichungsnummer: **0 248 767 B1**

(12) **EUROPÄISCHE PATENTSCHRIFT**

(45) Veröffentlichungstag der Patentschrift:
04.07.90

(51) Int. Cl.⁵: **G01N 3/30**

(21) Anmeldenummer: **87810322.5**

(22) Anmeldetag: **03.06.87**

(54) **Verfahren und Vorrichtung zum Spannungstesten von hängend angeordneten Glasbehältern.**

(30) Priorität: **03.06.86 CH 2247/86**

(43) Veröffentlichungstag der Anmeldung:
**09.12.87 Patentblatt 87/50**

(45) Bekanntmachung des Hinweises auf die Patenterteilung:
**04.07.90 Patentblatt 90/27**

(84) Benannte Vertragsstaaten:
**AT CH DE ES FR GB GR IT LI NL SE**

(56) Entgegenhaltungen:
**DE-C- 3 413 795**
**US-A- 4 363 649**

**PATENT ABSTRACTS OF JAPAN, Band 10,**
**Nr. 15 (P-422)[2072], 21. Januar 1986; & JP - A**
**- 60 171432 (ISHIZUKA GLASS K.K.) 04.09.1985**

(73) Patentinhaber: **ELPATRONIC AG, Baarerstrasse 117,**
**CH-6300 Zug(CH)**

(72) Erfinder: **Bogatzki, Hans-Ulrich,**
**Ferdinand-Hodler-Strasse 24, CH-8049 Zürich(CH)**

(74) Vertreter: **Hotz, Klaus, Dipl.-El.-Ing. / ETH, c/o**
**SOUDRONIC AG Industriestrasse 35 Postfach 11,**
**CH-8962 Bergdietikon(CH)**

# Beschreibung

Die Erfindung betrifft ein Verfahren zum Spannungstesten von hängend angeordneten Glasbehältern durch Impulsbeaufschlagung des Behälterbodens mittels eines Stößels. Außerdem betrifft die Erfindung eine Vorrichtung zur Durchführung des Verfahrens, mit einem Aufhängewerkzeug zum Erfassen des Glasbehälters nahe seiner Mündung und mit einer Einrichtung zum Ein- und Ausbringen des Stößels.

Glasbehälter sind aufgrund des amorphen Glasgefüges, aufgrund von Einschlüssen, aufgrund ihrer Behandlung bei der Herstellung, usw. nie frei von inneren Spannungen. Beim Befüllen oder Wiederbefüllen werden die Glasbehälter Beanspruchungen ausgesetzt, die zum Freisetzen dieser Spannungen und dadurch zum Bruch führen können. Da der Bruch der Glasbehälter beim Abfüller besonders unangenehm ist, werden Glasbehälter üblicherweise bereits in der Glashütte oder, im Falle des Wiederbefüllens von bereits ein- oder mehrmals verwendeten Glasbehältern, vor dem Wiederbefüllen einem Spannungstest, d.h. einer sogenannten Stress-Prüfung unterzogen.

Glasbehälter, die in der Glashütte als Massenprodukt nach dem Preß-Blas- oder dem Blas-Blas-Verfahren aus neuem Glaswerkstoff oder aus wieder eingeschmolzenen Altglasscherben hergestellt werden, sind nicht frei von inneren Spannungen, weil unvermeidlich ungleichmäßige Wanddicken und, besonders bei der Herstellung aus Altglasscherben, Einschlüsse vorhanden sind. Auch das Abkühlen führt unvermeidlich zu Spannungen. In dem Glasbehälter bilden sich Spannungsnester, die sich nicht sofort lösen, sondern erst bei Erschütterungen oder bei der Beanspruchung beim Befüllen zum Bruch führen können. Den gleichen Effekt können Haarrisse im Glas haben. In der Glashütte werden zwar Rißprüfer eingesetzt, um solche Haarrisse zu erkennen, durch die Rißprüfung können jedoch nicht alle Haarrisse erkannt werden, weil die Rißprüfer nur dort angesetzt werden, wo üblicherweise Risse auftreten, also z.B am Hals der Glasbehälter.

Bei der Verwendung von Altglasscherben zur Herstellung von Glasbehältern kommt es häufig vor, daß die Glasbehälter Keramikeinschlüsse und andere mögliche Restpartikel aufweisen, die mit eingeschmolzen worden sind. Um solche Einschlüsse oder "Steine" bilden sich besonders häufig Spannungsnester, die den Bruch des Glasbehälters verursachen können, wenn dieser Erschütterungen ausgesetzt wird, beispielsweise beim Befüllen.

Einwegglasbehälter verursachen hinsichtlich der Bruchgefahr Probleme, denn wenn sie nach der Herstellung in der Glashütte den dort durchgeführten Spannungstest bestanden haben, werden sie nur einmal befüllt und kommen dann als Altglas in die Glashütte zurück, um wieder eingeschmolzen zu werden. Anders ist es bei wiederverwendbaren Glasbehältern, die im Mittel bis zu zwanzigmal wiederbefüllt werden können. Selbst wenn solche Glasbehälter den ersten Spannungstest in der Glashütte bestanden und auch ein- oder mehrmaliges Wiederbefüllen überstanden haben, können die in ihnen nach wie vor enthaltenen Eigenspannungen irgendwann zum Bruch füh ren. Es ist klar, daß dieser Bruch beim Glashersteller wesentlich weniger problematisch als beim Abfüller ist. Glasformmaschinen arbeiten mit Produktionsgeschwindigkeiten von 300 bis 400 Glasbehältern/Stunde. Bis zu 3 Prozent der Glasbehälter gehen beim Spannungstest im Anschluß an ihre Herstellung zu Bruch und werden einfach wieder eingeschmolzen, was praktisch unproblematisch ist. Dagegen arbeiten Abfüller mit Produktionsgeschwindigkeiten von 60.000 bis 70.000 abgefüllten Glasbehältern pro Stunde. Es ist einleuchtend, daß in diesem Fall bereits eine Glasbruchrate von 1 oder 0,5 Promille eine erhebliche Produktionsbeeinträchtigung mit sich bringt, weil beim Glasbruch der Inhalt der Glasbehälter die Abfüllmaschine verschmutzt und umfängliche Reinigungsarbeiten erforderlich macht. Noch problematischer ist es, wenn der Glasbruch erst nach dem Befüllen auftritt, beispielsweise wenn die gefüllten Glasbehälter bereits in beträchtlicher Höhe auf einer Palette verpackt sind und ein gefüllter Glasbehälter in der obersten Etage auf der Palette zu Bruch geht.

Es ist daher zweckmässig, wiederverwendbare Glasbehälter vor jedem neuen Füllvorgang beim Abfüller einem Spannungstest zu unterziehen, um die geschilderten Probleme nach Möglichkeit auszuschließen.

Für den erwähnten Spannungstest werden in der Glashütte (hinter der Glasformmaschine und der Füllmaschine) Stressprüfmaschinen eingesetzt. Bei einer solchen Stressprüfmaschine, wie sie vom Erfinder bereits gebaut worden ist, ist ein Prüfkarussell vorgesehen, in das die von einem Förderband kommenden Glasbehälter der Reihe nach einlaufen und jeweils durch eine Zange am Hals erfasst werden. Die frei in den Zangen hängenden Glasbehälter durchlaufen das Prüfkarussell in einer Horizontalebene.

Parallel zu der Bahn der Glasbehälter ist im Prüfkarussell eine Kurvenbahn angeordnet, auf denen Wagen laufen, wobei jeder Zange ein Wagen zugeordnet ist. Jeder Wagen trägt einen vertikal angeordneten Stößel, dessen unteres Ende auf die Mitte der Glasbehältermündung gerichtet ist. Auf ihrem Weg auf der Kurvenbahn bewegen die Wagen die Stößel in die Glasbehälter, und, wenn das untere Ende des Stößels eine bestimmte Höhe über dem Behälterboden erreicht hat, wird ein Ausklinkmechanismus betätigt. Der ausgeklinkte Stößel fällt dann frei auf den Behälterboden und beaufschlagt diesen mit einem von der Fallhöhe und dem Stößelgewicht abhängigen Schlagimpuls. Anschließend werden die Stößel durch eine an jedem Wagen vorgesehene Einrichtung zum Ein- und Ausbringen des Stößels wieder erfaßt und aus den Glasbehältern herausgehoben, wenn sich der Wagen auf der Kurvenbahn wieder aufwärts bewegt, so daß die Glasbehälter das Prüfkarussell unbehindert wieder verlassen können. Das Stößelgewicht und die Fallhöhe sind variierbar, wodurch sich unterschiedliche Kräfte für unterschiedliche Glasbehälter (dünn- oder dickwandige, eng- oder weithalsige, usw.) ergeben, mit denen die geeigneten Prüfbelastungen hervorgerufen werden können. Durch das Einstellen dieser Parameter kann eine Qualitätsverbesserung in dem Sinn erreicht werden, daß beim Abfüller später weniger Bruch entsteht bzw., wenn der Abfüller mit der Prüfmaschine arbeitet, daß beim anschließenden Befüllen kein Bruch entsteht.

Der Prüfung mit dem auf den Glasbehälterboden fallenden Stößel liegt der Gedanke zugrunde, in dem Glasbehälter Schwingungen hervorzurufen, die dessen Eigenspannungen freisetzen, so daß es zum Glasbruch kommt oder nicht. Mit dieser Schlagprüfung werden also quasi Bedingungen oder Erschütterungen simuliert, wie sie beim Befüllen und Transport des Glasbehälters auftreten. Die Summe der in dem Glasbehälter vorhandenen Eigenspannungen wegen Haarrissen, Einschlüssen, ungleichmäßiger Abkühlung des Glasbehälters aufgrund ungleichmäßiger Wanddicke (der Hals ist üblicherweise wesentlich dünner als der Behälterboden) wird also bei die sem Spannungstest entweder freigesetzt und führt zum Bruch des Glasbehälters, oder aber nicht freigesetzt und der Glasbehälter bleibt ganz, wobei im letzteren Fall angenommen wird, daß er dann auch die Beanspruchung beim Befüllen oder Wiederbefüllen und beim Transport aushält.

Dieser bekannte Spannungstest (siehe z.B. die amerikanische Patentschrift US-A 4 363 649) ist in verschiedener Hinsicht verbesserungsbedürftig. Erstens, die Variationsmöglichkeiten hinsichtlich des Stößelgewichts (üblich ist z.B. ein Stößelgewicht von 0,5 bis 1 kp) und der Fallhöhe (üblich ist z.B. eine Fallhöhe von 80 bis 100 mm) sind zu gering. Es können nicht für 100 verschiedene zu prüfende Glasbehälter entsprechend viele verschiedene Stößelgewichte vorgesehen werden. Auch die Fallhöhe kann nicht beliebig verändert werden, weil in dem Prüfkarussell zeitlich und räumlich nur ein begrenzter Bereich vorhanden ist, in welchem die Stößel ausgeklinkt werden können. Zweitens, die Wahl von Fallhöhe und Stößelgewicht erfolgen empirisch und erfordern beim Einrichten der Prüfmaschine für jede neue Art von Glasbehälter einen großen Zeitaufwand. Wenn Stößelgewicht und/oder Fallhöhe zu niedrig angesetzt werden, wird die Gefahr, daß es später beim Abfüllen zum Glasbruch kommt, vergrößert. Werden sie zu hoch angesetzt, kann es bereits beim Spannungstest zu übermäßig viel Bruch und auch zu Halsabrissen der in den Zangen frei hängend angeordneten Glasbehälter kommen. Noch nachteiliger ist, daß es bei zu großem Stößelgewicht und/oder zu großer Fallhöhe auch vorkommen kann, daß kein Bruch auftritt, daß aber unbemerkt in den Glasbehältern durch den Spannungstest zusätzliche Spannungen aufgebaut werden, die die Bruchgefahr beim Abfüllen sogar noch vergrößern. Drittens, Glasformmaschinen arbeiten heute mit einer Produktionsgeschwindigkeit von ca. 300 bis 400 Glasbehältern/Minute, wogegen Stressprüfmaschinen der vom Erfinder gebauten Art nur Durchsätze von 100 bis 120 Glasbehältern/Minute erreichen. Dieses Problem läßt sich bei den vorhandenen Stressprüfmaschinen nicht beseiti gen, weil wie erwähnt für das Fallenlassen des Stößels nur ein begrenzter räumlicher und zeitlicher Bereich zur Verfügung steht und die Prüfkarusselle nicht beliebig groß ausgeführt werden können, und zwar sowohl aus Platz- als auch aus Kostengründen (jede zusätzliche Zange im Prüfkarussell bedeutet erhebliche zusätzliche Investitionskosten).

Aufgabe der Erfindung ist es, ein Verfahren zum Spannungstesten von hängend angeordneten Glasbehältern durch Impulsbeaufschlagung des Behälterbodens mittels eines Stößels so zu verbessern, daß die Impulsbeaufschlagung auf einfache Weise ohne zusätzlichen Raum- und Zeitbedarf variiert und der Durchsatz an zu testenden Glasbehältern der Produktionsgeschwindigkeit vorhandener Glasformmaschinen angepaßt werden kann; außerdem soll eine Vorrichtung zur Durchführung des Verfahrens geschaffen werden.

Diese Aufgabe wird erfindungsgemäß dadurch gelöst, daß der Stößel auf dem Behälterboden abgesetzt wird und dann die Impulsbeaufschlagung durch einstellbare Krafteinwirkung auf den auf dem Behälterboden ruhenden Stößel erfolgt.

Die Vorrichtung zur Durchführung dieses Verfahrens ist erfindungsgemäß durch eine Einrichtung zum Absetzen des Stössels auf dem Behälterboden und durch eine im Bereich des vom Behälterboden abgewandten Endes des Stößels angeordnete, in ihrer Wirkung einstellbare Kraftquelle gekennzeichnet.

Vorteilhafte Ausgestaltungen der Erfindung bilden den Gegenstand der Unteransprüche.

Durch das Verfahren nach der Erfindung entfällt die beim herkömmlichen Spannungstesten vorhandene räumliche und zeitliche Beschränkung für die Impulsbeaufschlagung aufgrund der nur in begrenztem Maße variierbaren Parameter Stößelgewicht und Fallhöhe, denn die Impulsbeaufschlagung erfolgt erfindungsgemäß erst, nachdem der Stößel bereits auf den Behälterboden aufgesetzt worden ist. In einem Prüfkarussell kann das Aufsetzen lange vor dem Zeitpunkt der Impulsbeaufschlagung erfolgen, so daß für die Impulsbeaufschlagung selbst ausreichend Raum und Zeit verfügbar sind, um den Stößel gegebenenfalls mehreren Impulsbeaufschlagungen auszusetzen. Da erfindungsgemäß der bereits auf dem Behälterboden ruhende Stößel der Impulsbeaufschlagung ausgesetzt wird, bestehen zahlreiche Möglichkeiten der Impulserzeugung, wie sie zum Teil in den Unteransprüchen beansprucht sind. Erfindungsgemäß lassen sich die Impulsamplitude und/oder die Impulsfrequenz auf mechanischem, elektrischem oder ähnlichem Wege beliebig variieren, um den Glasbehälter mit einem dem Optimum möglichst nahekommenden Impuls beaufschlagen zu können. Bei dem herkömmlichen Spannungstest kann durch den einmaligen Fall des Stößels auf den Behälterboden nur eine abklingende Schwingung in dem Glasbehälter erzeugt werden, wogegen durch das Verfahren nach der Erfindung die Form und die Dauer der durch die Impulsbeaufschlagung in dem Glasbehälter erzeugten Schwingung beliebig variiert werden können, um dem Optimum für den Spannungstest möglichst nahezukommen. Während beim herkömmlichen Spannungstest nur ein einmaliger Schlag aufgrund einer voreingestellten Fallhöhe erzeugt werden kann, ist erfindungsgemäß die Impulsbeaufschlagung so variabel, daß Prallhöhen zwischen Stößel und Behälterboden erzeugt werden können, die ganz feine Schwingungen im Glasbehälter erzeugen. Außerdem stehen wie erwähnt genügend Raum und Zeit zur Verfügung, um die Impulsbeaufschlagung nötigerweise beliebig oft zu wiederholen. Ferner läßt sich durch das Verfahren nach der Erfindung der Glasbehälterdurchsatz beim Spannungstesten ohne weiteres auf Werte erhöhen, die der Produktionsgeschwindigkeit einer Glasformmaschine entsprechen, so daß wesentliche Zeit-, Raum- und Kostenvorteile erreicht werden.

Die Vorrichtung zur Durchführung des Verfahrens nach der Erfindung unterscheidet sich von herkömmlichen Vorrichtungen dieser Art im wesentlichen dadurch, daß eine Einrichtung zum Absetzen des Stößels auf dem Behälterboden (statt ihn auf den Behälterboden fallenzulassen) vorgesehen ist und daß im Bereich des vom Behälterboden abgewandten Endes des auf dem Behälterboden ruhenden Stößels eine einstellbare Kraftquelle angeordnet ist, bei der es sich um irgendeinen einstellbaren Impulserzeuger handeln kann.

In der Ausgestaltung der Erfindung nach Anspruch 2 ist die Krafteinwirkung stufenlos einstellbar, was bei einer magnetischen oder elektromagnetischen Kraftquelle also mittels eines Potentiometers erfolgen kann.

In der Ausgestaltung der Erfindung nach den Ansprüchen 3 und 9 können Schläge auf das vom Behälterboden abgewandte Ende des auf dem Behälterboden ruhenden Stößels ausgeübt werden, vorzugsweise mittels eines drehbaren Exzenters mit einstellbarer Exzentrizität.

In der Ausgestaltung der Erfindung nach den Ansprüchen 4 und 10 erfolgt die Impulsbeaufschlagung durch Ultraschalleinwirkung, also vorzugsweise mittels eines Ultraschallsenders. Die Glasbehälter bewegen sich in diesem Fall kontinuierlich an dem Ultraschallsender vorbei, der die auf dem Behälterboden ruhenden Stößel ein- oder mehrmals beschallt. Die Stößel werden dadurch impulsweise in Ultraschallschwingungen versetzt, die sie auf den Behälterboden und über diesen auf den gesamten Glasbehälter übertragen.

In der Ausgestaltung der Erfindung nach den Ansprüchen 5 und 11 kann die Kraftquelle aus einem magnetischen Stellantrieb bestehen, der durch eine Magnetkraft auf die Stößel einwirkt. Es kann sich einfach um eine Magnetspule mit festem Kern handeln, die den Stößel um eine minimale Höhe anhebt und wieder nach unten treibt, oder aber um einen Stellantrieb, bei dem ein Magnetanker unmittelbar auf das obere Stößelende mechanisch einwirkt.

In der Ausgestaltung der Erfindung nach den Ansprüchen 6 und 12 erfolgt die Impulsbeaufschlagung der Stößel wieder rein mechanisch, beispielsweise mittels einer Kufe, die auf die vom Behälterboden abgewandten Enden der Stößel direkt einwirkt und durch eine Vorrichtung ähnlich wie bei einem Vibrationstisch in Vibration versetzt wird.

In der Ausgestaltung der Erfindung nach den Ansprüchen 7, 13 und 14 werden Stößel geringer Masse eingesetzt, also vorzugsweise in Form eines Hohlzylinders oder aus verstärktem Kunststoff. Ein solcher Stößel geringer Masse kann hochfrequent in Schwingung versetzt werden, wodurch sich schwere und massive Stöße auf einfache Weise vermeiden lassen. Es dürfte auch wesentlich günstiger sein, Schwingungen in dem Glasbehälter mit einer kleinen Masse hochfrequent auszulösen statt mit einer großen Masse niederfrequent wie bei dem herkömmlichen Spannungstestverfahren.

Mehrere Ausführungsbeispiele der Erfindung werden im folgenden unter Bezugnahme auf die Zeichnungen näher beschrieben.

Es zeigt

Fig. 1 eine Ausführungsform der Vorrichtung zur Durchführung des Verfahrens nach der Erfindung mit einem drehbaren Exzenter,

Fig. 2 eine weitere Ausführungsform der Vorrichtung mit einem Ultraschallsender und

Fig. 3 noch eine weitere Ausführungsform der Vorrichtung mit einem magnetischen Stellantrieb.

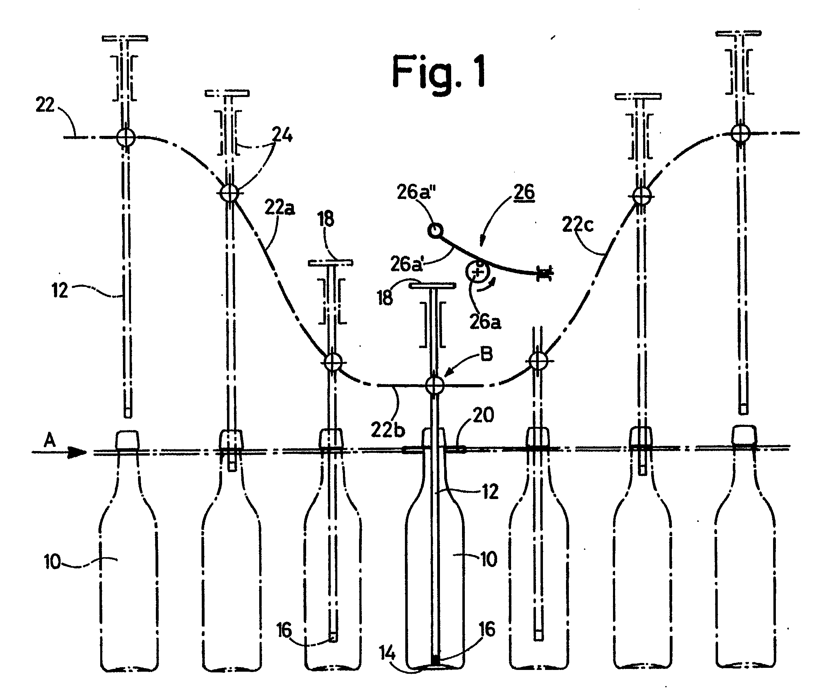

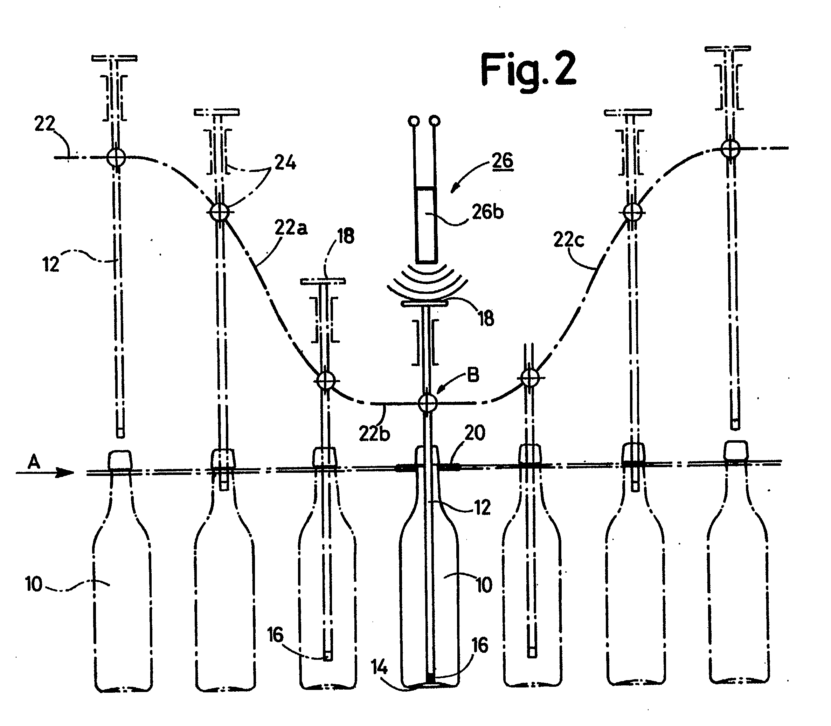

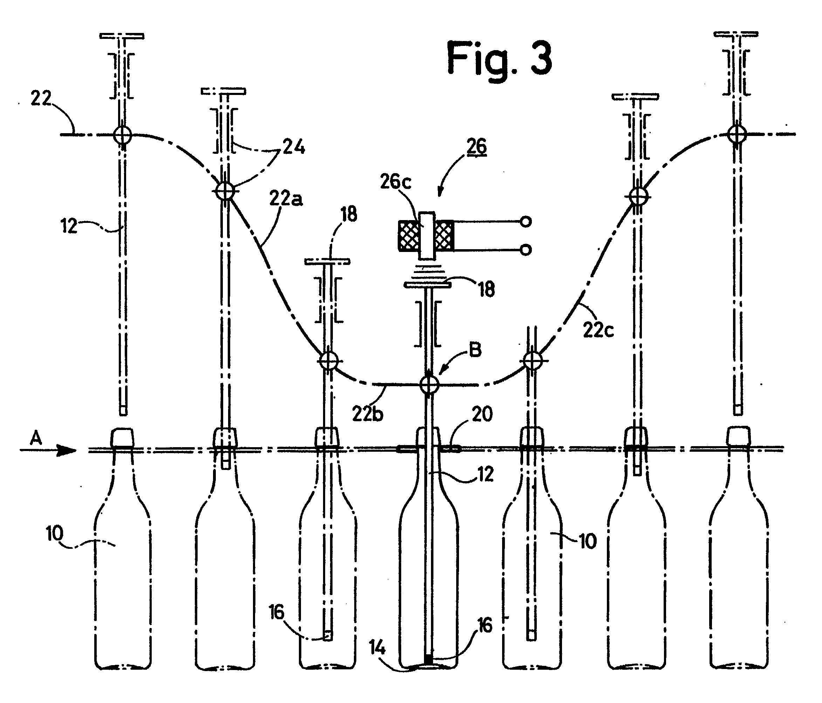

Fig. 1 zeigt den für die Erfindung wesentlichen Teil einer Ausführungsform einer Vorrichtung zum Spannungstesten von hängend angeordneten Glasbehältern 10. Die dargestellte Vorrichtung kann als ein in die Ebene abgewickelter Teil des Prüfkarussells einer Stressprüfmaschine aufgefaßt werden, die aber erfindungsgemäß dahingehend abgewandelt worden ist, daß ihre Stößel 12 nicht mehr ausgeklinkt und auf den Behälterboden 14 fallengelassen werden, sondern vielmehr sanft mit ihrem unteren Ende 16 auf den Behälterboden 14 aufgesetzt und dann an ihrem oberen Ende 18 einer einstellbaren Krafteinwirkung ausgesetzt werden. Die Glasbehälter 10 laufen in Richtung des Pfeils A von einem nicht dargestellten Förderer in die Vorrichtung ein, in der sie durch Zangen 20 am Hals hängend aufgenommen werden. Benachbart zu der Bahn der Zangen 20 ist eine Kurvenbahn 22 angeordnet, auf der in festen, dem gegenseitigen festen Mittenabstand der in den Zangen 20 hängenden Glasbehälter 10 entsprechenden Abständen laufende Wagen 24 angeordnet sind. Die Wagen 24, die sich auf der Kurvenbahn 22 in Fig. 1 nach rechts bewegen, bewegen sich auf einem abfallenden Teil 22a der Kurvenbahn 22 nach unten, wobei die Stößel 12 in die Glasbehältermündungen eingeführt und ihre unteren Enden 16 den Behälterböden 14 genähert werden. Wenn ein Wagen 24 einen horizontalen Teil 22b der Kurvenbahn 22 erreicht, setzt er den Stößel 12 auf den Behälterboden 14 auf. Im gesamten Bereich des horizontalen Teils 22b der Kurvenbahn 22 ist die Krafteinwirkung auf das obere Stößelende 18 möglich. Anschließend an den horizontalen Teil 22b der Kurvenbahn 22 bewegen sich die Wagen 24 auf einem ansteigenden Teil 22c der Kurvenbahn wieder nach oben und ziehen dabei die Stößel aus den Glasbehältern 10 heraus. In der mit B bezeichneten Mitte des horizontalen Teils 22b der Kurvenbahn 22 ist dem oberen Ende 18 des Stößels 12 eine in ihrer Wirkung einstellbare Kraftquelle 26 zugeordnet, die bei dem Ausführungsbeispiel nach Fig. 1 einen Exzenter 26a, bei dem Ausführungsbeispiel nach Fig. 2 einen Ultraschallsender 26b und bei dem Ausführungsbeispiel nach Fig. 3 einen magnetischen Stellantrieb 26c aufweist, die im folgenden im einzelnen beschrieben sind.

Bei dem Ausführungsbeispiel nach Fig. 1 ist dem Exzenter 26a ein biegsamer Arm 26a' zugeordnet, der am rechten Ende eingespannt ist und am linken Ende eine Kugel 26a'' trägt, die bei jeder Umdrehung des Exzenters 21a einmal auf das obere Ende 18 des Stößels 12 schlägt. In allen hier dargestellten Ausführungsbeispielen ist das obere Ende 18 des Stößels 12 als Teller ausgebildet, was aber lediglich eine bevorzugte und nicht notwendige Ausführungsform dieses Endes darstellt. Mittels der Drehzahl des Exzenters 26a und/oder der Exzentrizität läßt sich die Krafteinwirkung auf den Stößel 12 stufenlos einstellen. Auch die dargestellte Ausführungsform des Exzenters 26a dient lediglich zur Veranschaulichung, denn statt dessen könnte auch eine Nockenscheibe benutzt werden, die direkt auf das obere Ende 18 des Stößels 12 einwirkt. Bei dem Ausführungsbeispiel nach Fig. 1 kann der Stößel 12 als Hohlzylinder aufgebaut sein, der an seinem unteren Ende 16 mit einem Druckstück, z.B. aus Kunststoff, versehen ist. Statt dessen kann der Stößel 12 auch einfach ein Stab aus verstärktem Kunststoff sein. In jedem Fall kann der Stößel gegenüber dem bei der bekannten Stressprüfmaschine verwendeten Stößel eine wesentlich geringere Masse aufweisen, da statt der herkömmlicherweise ausgenutzten Fallbeschleunigung des Stößels zur Schlagerzeugung erfindungsgemäß lediglich impulsweise auf den auf dem Behälterboden 14 ruhenden Stößel 12 eingewirkt wird. Wenn andere Glasbehälter 10 zu testen sind, braucht nicht der Stößel 12, sondern lediglich die Krafteinwirkung auf diesen geändert zu werden.

Das Ausführungsbeispiel nach Fig. 2 unterscheidet sich von dem Ausführungsbeispiel nach Fig. 1 lediglich dadurch, daß die Kraftquelle 26 statt des Exzenters den Ultraschallsender 26b aufweist. Sämtliche Ausführungen zu dem Ausführungsbeispiel nach Fig. 1 gelten daher entsprechend für das Ausführungsbeispiel nach Fig. 2. Der Ultraschallsender 26b versetzt den Stößel 12 in Ultraschallschwingungen, die dieser auf den Behälterboden 14 überträgt. Der Ultraschallsender 26b kann ein herkömmlicher piezoelektrischer Schwingquarz sein, dessen Schwingungsamplitude und/oder -frequenz auf übliche Weise einstellbar sind.

In dem Ausführungsbeispiel nach Fig. 3 weist die Kraftquelle 26 einen magnetischen Stellantrieb 26c auf. Dieser ist als eine Magnetspule mit festem Kern dargestellt, der den am oberen Ende 18 des Stößels vorgesehenen Teller, der aus Eisen od. dgl. besteht, mit der Frequenz der Erregerspannung des Magnets anzieht und abstößt. Durch die Dauer des Anlegens der Erregerspannung läßt sich die Impulslänge auf einfache Weise einstellen. Außer dem Teller könnte auch der gesamte übrige Stößel 12 aus Eisen od. dgl. bestehen. Ein Anker des magnetischen Stellantriebs könnte auch direkt auf den Teller am oberen Ende 18 aufgesetzt werden und axial beweglich ausgebildet sein (nicht dargestellt), wobei dann die Schwingungsbewegung des Ankers direkt auf den Stößel 12 übertragen würde. In diesem Fall bräuchte der Teller und/oder der Stößel selbstverständlich nicht aus Eisen od. dgl. zu bestehen, sondern könnte wie in den beiden anderen Ausführungsbeispielen auch aus verstärktem Kunststoff bestehen.

Bei allen drei dargestellten Ausführungsbeispielen kann der horizontale Teil 22b der Kurvenbahn 22 beliebig lang ausgebildet und die Kraftquelle 26 auf einem Teil des Weges mit dem auf dem Behälterboden 14 ruhenden Stößel 12 mitgeführt werden, so daß beliebig Zeit für mehrere Krafteinwirkungen auf diesen Stößel zur Verfügung steht.

Statt der in den Fig. 1 bis 3 dargestellten verschiedenen drei Kraftquellen könnte auch eine Kufe benutzt werden (nicht dargestellt), die an einem Ende direkt auf den Teller des auf dem Behälterboden 14 ruhenden Stößels 12 einwirkt und am anderen Ende durch eine Antriebsvorrichtung wie bei einem Vibrationstisch in Vibration versetzt wird. Die Impulslänge der Krafteinwirkung könnte dabei durch die Kufenlänge im Bereich der Berührung mit dem Teller oder durch die Vibrationsdauer der Kufe eingestellt werden.

**Patentansprüche**

1. Verfahren zum Spannungstesten von hängend angeordneten Glasbehältern durch Impulsbeaufschlagung des Behälterbodens mittels eines Stößels, **dadurch gekennzeichnet**, daß der Stößel auf dem Behälterboden abgesetzt wird und dann die Impulsbeaufschlagung durch einstellbare Krafteinwirkung auf den auf dem Behälterboden ruhenden Stößel erfolgt.

2. Verfahren nach Anspruch 1, dadurch gekennzeichnet, daß die Krafteinwirkung nach Größe und/oder Dauer stufenlos eingestellt wird.

3. Verfahren nach Anspruch 1 oder 2, dadurch gekennzeichnet, daß die Krafteinwirkung durch einen oder mehrere Schläge auf das vom Behälterboden abgewandte Ende des Stößels erzeugt wird.

4. Verfahren nach Anspruch 1 oder 2, dadurch gekennzeichnet, daß die Krafteinwirkung durch Ultraschalleinwirkung auf das vom Behälterboden abgewandte Ende des Stößels erzeugt wird.

5. Verfahren nach Anspruch 1 oder 2, dadurch gekennzeichnet, daß die Krafteinwirkung durch Magnetkrafteinwirkung auf das vom Behälterboden abgewandte Ende des Stößels erzeugt wird.

6. Verfahren nach Anspruch 1 oder 2, dadurch gekennzeichnet, daß die Krafteinwirkung durch auf das vom Behälterboden abgewandte Ende des Stößels direkt einwirkende, wie bei einem Vibrationstisch erzeugte mechanische Schwingungen erzeugt wird.

7. Verfahren nach einem der Ansprüche 1 bis 6, dadurch gekennzeichnet, daß ein Stößel geringer Masse eingesetzt wird.

8. Vorrichtung zur Durchführung des Verfahrens nach Anspruch 1, mit einem Aufhängewerkzeug zum Erfassen eines Glasbehälters nahe seiner Mündung und mit einer Einrichtung zum Ein- und Ausbringen eines Stößels, **gekennzeichnet** durch eine Einrichtung (22, 24) zum Absetzen eines Stößels (12) auf dem Behälterboden (14) und durch eine im Bereich des vom Behälterboden abgewandten Endes (18) des Stößels (12) angeordnete, in ihrer Wirkung einstellbare Kraftquelle (26).

9. Vorrichtung nach Anspruch 8, dadurch gekennzeichnet, daß die Kraftquelle einen auf das vom Behälterboden (14) abgewandte Ende (18) des Stößels (12) einwirkenden drehbaren Exzenter (26a) mit einstellbarer Exzentrizität aufweist.

10. Vorrichtung nach Anspruch 8, dadurch gekennzeichnet, daß die Kraftquelle (26) einen Ultraschallsender (26b) aufweist.

11. Vorrichtung nach Anspruch 8, dadurch gekennzeichnet, daß die Kraftquelle (26) einen magnetischen Stellantrieb (26c) aufweist.

12. Vorrichtung nach Anspruch 8, dadurch gekennzeichnet, daß die Kraftquelle (26) eine nach dem Vibrationstischprinzip aufgebaute Einrichtung ist.

13. Vorrichtung nach einem der Ansprüche 8 bis 12, dadurch gekennzeichnet, daß der Stößel (12) ein Hohlzylinder ist, der an seinem dem Behälterboden (14) benachbarten Ende (16) mit einem Druckstück versehen ist.

14. Vorrichtung nach einem der Ansprüche 8 bis 13, dadurch gekennzeichnet, daß der Stößel (12) wenigstens zum Teil aus verstärktem Kunststoff besteht.

## Claims

1. A method for the stress testing of glass containers, which are arranged hanging, by impulse action on the bottom of the container by means of an impacting rod, characterised in that the impacting rod is placed down on the bottom of the container and then the impulse action is effected by an adjustable action of force on the impacting rod resting on the bottom of the container.

2. A method according to Claim 1, characterised in that the action of force is adjusted in a continuously variable manner in magnitude and/or duration.

3. A method according to Claim 1 or 2, characterised in that the action of force is produced by one or more blows on the end of the impacting rod remote from the bottom of the container.

4. A method according to Claim 1 or 2, characterised in that the action of force is produced by ultrasonic action on the end of the impacting rod remote from the bottom of the container.

5. A method according to Claim 1 or 2, characterised in that the action of force is produced by the action of magnetic force on the end of the impacting rod remote from the bottom of the container.

6. A method according to Claim 1 or 2, characterised in that the action of force is produced by mechanical vibrations, produced as in the case of a vibratory table and acting directly on the end of the impacting rod remote from the bottom of the container.

7. A method according to any one of Claims 1 to 6, characterised in that an impacting rod of small mass is used.

8. An apparatus for carrying out the method according to Claim 1, having a suspension appliance to grasp a glass container near its mouth and having a device for introducing and extracting an impacting rod, characterised by a device (22, 24) for placing the impacting rod (12) down on the bottom (14) of the container and by a source of energy (26) which is disposed in the region of the end (18) of the impacting rod (12) remote from the bottom of the container and is adjustable in its action.

9. An apparatus according to Claim 8, characterised in that the source of energy comprises a rotatable eccentric (26a) with adjustable eccentricity, acting on the end (18) of the impacting rod (12) remote from the bottom (14) of the container.

10. An apparatus according to Claim 8, characterised in that the source of energy (26) comprises an ultrasonic transmitter (26b).

11. An apparatus according to Claim 8, characterised in that the source of energy (26) comprises a magnetic actuating drive (26c).

12. An apparatus according to Claim 8, characterised in that the source of energy (26) is a device constructed on the vibratory table principle.

13. An apparatus according to any one of Claims 8 to 12, characterised in that the impacting rod (12) is a hollow cylinder which is provided with a pressure member at its end (16) adjacent to the bottom (14) of the container.

14. An apparatus according to any one of Claims 8 to 13, characterised in that the impacting rod (12) consists at least partially of reinforced plastics material.

## Revendications

1. Procédé pour des essais en tension de récipients en verre suspendus, par impulsions transmises au fond du récipient à l'aide d'un mouton, caractérisé en ce que le mouton est abaissé sur le fond du récipient et en ce que les impulsions sont ensuite transmises par une force réglable agissant sur le mouton reposant sur le fond du récipient.

2. Procédé selon la revendication 1, caractérisé en ce que les effets de la force sont réglés en continu, en grandeur et/ou durée.

3. Procédé selon la revendication 1 ou 2, caractérisé en ce que la force agit par un ou plusieurs chocs produits sur l'extrémité du mouton, éloignée du fond du récipient.

4. Procédé selon la revendication 1 ou 2, caractérisé en ce que la force agit par des ultra-sons produits sur l'extrémité du mouton éloignée du fond du récipient.

5. Procédé selon la revendication 1 ou 2, caractérisé en ce que la force agit par effet magnétique sur l'extrémité du mouton éloignée du fond du récipient.

6. Procédé selon la revendication 1 ou 2, caractérisé en ce que la force agit par des oscillations mécaniques produites sur l'extrémité du mouton éloignée du fond du récipient, agissant directement comme avec une table vibrante.

7. Procédé selon l'une des revendications 1 à 6, caractérisé en ce qu'on utilise un mouton de masse réduite.

8. Dispositif pour la mise en œuvre du procédé selon la revendication 1, comportant un outil suspendu destiné à saisir un récipient en verre à proximité de son embouchure, et comportant un dispositif d'introduction et d'extraction d'un mouton, caractérisé par un dispositif (22, 24) destiné à abaisser le mouton (12) sur le fond du récipient (14), et par une source d'énergie (26) d'effet réglable, située dans la zone de l'extrémité (18) du mouton (12), éloignée du fond du récipient.

9. Dispositif selon la revendication 8, caractérisé en ce que la source d'énergie comporte un excentrique (26a) tournant, d'excentricité réglable, agissant sur l'extrémité (18) du mouton (12), éloignée du fond du récipient (14).

10. Dispositif selon la revendication 8, caractérisé en ce que la source d'énergie (26) comporte un émetteur d'ultra-sons (26b).

11. Dispositif selon la revendication 8, caractérisé en ce que la source d'énergie (26) comporte un actionneur (26c) magnétique.

12. Dispositif selon la revendication 8, caractérisé en ce que la source d'énergie (26) est un dispositif conçu selon le principe d'une table vibrante.

13. Dispositif selon l'une des revendications 8 à 12, caractérisé en ce que le mouton (12) est un cylindre creux qui est pourvu d'une pièce de pression à son extrémité (16) voisine du fond du récipient (14).

14. Dispositif selon l'une des revendications 8 à 13, caractérisé en ce que le mouton (12) est réalisé au moins en partie en matière synthétique.

Fig. 1

EP 0 248 767 B1

Fig.2

Fig. 3